# EUROPEAN PATENT APPLICATION

(11) **EP 2 384 769 A2**
(43) Date of publication of application: **09.11.2011**
(21) Application number: 11173005.7
(22) Date of filing: 14.03.2007
(51) Int. Cl.: A61K 49/00, A61K 9/00

(54) **Ophthalmic visualization compositions and methods of using same**

(30) Priority: 17.03.2006 US 378506
(62) Divisional of application: 07853475.7
(71) Applicant: Allergan, Inc., Irvine, CA 92612 (US)
(72) Inventor: Chang, James N., Newport Beach, CA California 92660 (US); Lyons, Robert T., Laguna Hills, CA California 92653 (US)
(74) Representative: HOFFMANN EITLE

(57) **Abstract**

Compositions and methods useful for at least assisting in visualizing vitreous bodies of eyes of humans or animals are provided. Such compositions include a contrast component and a carrier component and are substantially free of any therapeutic agent effective to have pharmacological activity in an eye in which the composition is placed. Methods of using such compositions as visualization devices are also provided.

## Description

This application claims priority pursuant to 35 U.S.C. Section 110(e) to United States patent application Serial No. 11/378,506 filed on March 17, 2006, which is hereby incorporated by reference in its entirety.

### Background of Invention

The present invention relates to ophthalmic compositions useful as visualization devices and methods of using such compositions. More particularly, the invention relates the ophthalmic compositions useful to facilitate visualization of the vitreous cavity or vitreous body of an eye, for example, during vitrectomy surgery, when placed in the vitreous of an eye, and to methods of using such compositions.

Commercial triamcinolone acetonide suspensions, for example, a formulation identified as Kenalog® 40, have been used to help visualize the vitreous body during eye surgery. For example, intracameral triamcinolone acetonide is helpful for visualizing the vitreous body in the anterior chamber during cataract surgery and for detecting undetached cortical vitreous after posterior vitreous detachment. Each milliliter (ml) of the Kenalog® 40 composition includes 40 milligrams (mg) of triamcinolone acetonide (TA), sodium chloride as a tonicity agent, 10 mg of benzyl alcohol as a preservative, and 7.5 mg of carboxymethylcellulose and 0.4 mg of polysorbate 80 as resuspension aids. Although widely used by ophthalmologists, this commercially available formulation suffers from several important limitations.

For example, there are significant safety concerns about using a potent steroid, TA, as a visualization device. These concerns include the possibility of an increase in intraocular pressure, cataract formation or sterile endophthalmitis induced by the benzyl alcohol preservative present in the Kenalog® 40 formulation.

There is a need for new compositions for placement in eyes to facilitate visualization of the vitreous body, for example, during surgery.

### Summary of the Invention

New compositions and methods useful for at least assisting in visualizing vitreous bodies of eyes of humans or animals have been discovered. The present compositions are highly suitable for intravitreal placement, for example, to facilitate or enhance visualization, with reduced risk of ocular, for example, lens, vitreal and retinal, side effects when used in an eye. The present compositions are effective as visualization devices and to not cause or have side effects that often occur when formulations including potent therapeutic agents, such as in Kenalog® 40, are used for visualization purposes. Overall, the present compositions are easily and effectively administerable, for example, injectable, into the vitreous of an eye of a human or animal, and provide very effective visualization of the vitreous body, for example, during surgery.

In one broad aspect, the present invention is directed to ophthalmic compositions effective as visualization devices in the vitreous, for example during surgery, such as vitrectomy surgery. The present compositions comprise a contrast component in an amount effective when a composition is placed in a vitreous of an eye (1) to be opaque, for example to light, such as visible light, or (2) to be visually distinct, for example, to the vitreous of the eye, for example, in light, such as visible light; and a carrier component combined with the contrast component. The carrier component is effective to act as a carrier for the contrast component.

The present compositions are advantageously substantially free of any therapeutic agent effective to have pharmacological activity in an eye in which the composition is placed.

Preferably, the compositions are substantially free of added preservative components. The present compositions advantageously contain no benzyl alcohol preservative which may cause unwanted side effects or reactions when placed in an eye.

In one embodiment, the present compositions are provided such that the contrast component and the carrier component have different refractive indexes, for example refractive indexes which differ by at least about 0.05 and advantageously by at least about 0.15.

Without wishing to limit the invention to any particular theory of operation, it is believed that the difference in refractive index between the contrast component and the carrier component is useful in providing the present compositions with a degree of opacity or visual distinctiveness effective as a visualization device for the vitreous body in accordance with the present invention.

The contrast component may be soluble or insoluble in the composition, for example, at conditions inside or outside the eye. Preferably, the contrast component is substantially non-toxic in the eye in which composition is placed. In one useful embodiment, the contrast component comprises a plurality of particles.

The contrast component may be inorganic or organic, biodegradable or non-biodegradable.

Examples of inorganic contrast components include, without limitation, talc, titanium oxide, zinc oxide and the like and mixtures thereof.

In one useful embodiment, the contrast component comprises a polymeric material, for example, a polymeric material which comprises a plurality of particles. The polymeric material may be selected from homopolymers, copolymers, that is polymers which include units from 2 or more monomers, and mixtures thereof.

Examples of biodegradable polymeric materials which may be employed in the present contrast components include, without limitation, those selected from polylactides, polglycolides, poly(lactide-coglycolides), polyanhydrides, poly(caprolactone), polycarbonates, polyarylates, polydioxanones, polyhydroxyalkanoates, polyphosphazenes, chitosan and the like and mixtures thereof.

Non-biodegradable polymeric materials useful in the present invention include, without limitation, polyolefins, ethylene vinyl acetate copolymers, silicone polymer, for Example, polysiloxanes and the like, poly(vinyl chloride) and the like and mixtures thereof,

The carrier component preferably is substantially non-toxic in an eye in which the composition is placed. Preferably, the carrier component is ophthalmically acceptable in an eye in which the composition is placed.

In one very useful embodiment, the carrier component is aqueous or aqueous-based, for example, and without limitation, the carrier component may comprise a major amount, that is at least about 50% w/v, of water.

In one embodiment, the contrast component and the carrier component together form an opaque solution or a colored solution. Thus, although the present compositions are very useful as suspensions of the contrast component in the carrier component, in certain embodiments the contrast component is soluble in the carrier component and forms an opaque or colored solution which is useful as a visualization device in accordance with the present invention. Such solutions may be advantageous in that no resuspension components are included since the contrast component is in solution.

In one embodiment, the present compositions comprise oil-in-water emulsions, for example, submicron oil-in-water emulsions. Such emulsions are highly stable, advantageously biodegradable, and have sufficient opacity/visual distinctiveness to be effective visualization devices or aids in accordance with the present invention. Any suitable oil or combination of oils may be employed in the present compositions provided that such oils cause no significant or undue harm to the eye in which the oil-containing compositions or emulsions are placed.

The carrier component may include at least one additional component in an amount effective to enhance the ocular compatibility or ophthalmic acceptability of the composition relative to an identical composition without the at least one additional component. For example, in one very useful embodiment, the carrier component is aqueous and the at least one additional component is selected from buffer component, tonicity components, resuspension components, emulsification components and the like and mixtures thereof.

One very useful form for the present compositions is as an aqueous suspension, that is compositions comprising a plurality of particles of contrast component, and an aqueous, such as a liquid aqueous, carrier component.

The carrier component may also include a resuspension component in an amount effective to provide enhanced suspension maintenance to the composition or to provide enhanced stability to the suspension to reform as a composition relative to an identical composition without the resuspension component. Examples of resuspension components include, without limitation, cellulose or cellulosic components, surfactant components, wettability components and the like and mixtures thereof.

In one embodiment, the present compositions are sterile. For example, the present compositions may be sterilized, such as by conventional sterilizing techniques, before and/or daring and/or after being packaged.

The present compositions advantageously include no preservative component, for example, no added preservative component. The present compositions may be advantageously provided in bulk form. However, particularly with regard to the use of the present compositions in surgical procedures, the present compositions are advantageously provided, for example, packaged, in a single use format.

The viscosity of the present compositions may vary over a relatively wide range provided that such viscosity does not substantially or significantly interfere with the usefulness of the composition as a visualization device as described herein. In one embodiment, the present compositions have viscosities of about 50 cps or less, or about 30 cps or less, or about 20 cps or less. Viscosities in a range of about 1 cps to about 3 cps or about 5 cps or about 10 cps are very useful. Relatively low viscosities are useful in facilitating administering, for example, injecting, the compositions into the vitreous and/or facilitating the compositions dispersing in the vitreous body to provide better or more complete visualization of the vitreous.

Methods of using the present compositions are provided and are included within the scope of the present invention.

In one embodiment, methods for providing visualization of a vitreous body of an eye are provided. Such methods comprise placing an effective amount of a composition comprising a contrast component and a carrier component, as described elsewhere herein, in a vitreous body of an eye.

In a further aspect of the present invention, methods are provided for removing at least a portion of the vitreous from an eye. Such methods comprise placing an effective amount of a composition comprising a contrast component and a carrier component, as described elsewhere herein, in a vitreous body of an eye; and, thereafter, removing at least a portion of the vitreous from the eye.

Each and every feature described herein, and each and every combination of two or more of such features, is included within the scope of the present invention provided that the features included in such a combination are not mutually inconsistent. In addition, any feature or combination of features may be specifically excluded from any embodiment of the present invention.

These and other aspects and advantages of the present invention are apparent in the following detailed description, examples and claims of the present application.

### Detailed Description

The present invention involves compositions effective as visualization devices in a vitreous of an eye, such as when placed in, e.g., injected in, a vitreous of an eye of a human or animal. Such compositions in the vitreous of the eye are effective to allow visualization, such as direct visualization, for example, under visible light, of the vitreous body by a surgeon performing a surgical procedure, for example, a surgical vitrectomy, on the eye. Such visualization of the vitreous body is highly advantageous, for example, in that it allows the surgeon to see the extent of the vitreous body and to perform the surgical procedure or operation more effectively and more safely.

In one broad aspect of the present invention, the present compositions comprise a contrast component and a carrier component. The compositions are advantageously ophthalmically acceptable.

The present compositions are preferably more compatible with or more "friendly" to the eye, for example, to the tissues in the posterior segment of the eye, such as, to the tissues of the retina of the eye, relative to compositions previously used as vitreous body visualization devices or aids, for example, the composition sold under the trademark Kenalog® 40 by Bristol-Myers Squibb. In particular, the present compositions are substantially free of any therapeutic component effective to have pharmacological activity in an eye in which such compositions are placed. For example, the present compositions are substantially free of any corticosteroid component, such as is present in Kenalog® 40. Advantageously, the present compositions are substantially free of added preservative components or include effective preservative components which are more compatible with or more "friendly" to the posterior segment, e.g., retina, of the eye relative to benzyl alcohol, which is included in the Kenalog® 40 composition as a preservative.

In one aspect, the present invention is directed to compositions which are structured to be visualization devices and which have substantially no other therapeutic effect and/or have substantially no therapeutic component which has pharmacological activity in an eye in which the compositions are placed. In one embodiment, the present compositions may be considered to be specifically or custom formulated as visualization devices which are designed to be compatible or more "friendly" to the eye, for example, to the human eye. This is in contrast to prior art compositions used as visualization devices, such as Kenalog® 40, which include potent drugs and/or relatively eye unfriendly preservatives, which can have one or more unwanted effects on the eye.

The present compositions are relatively straightforward and are designed as visualization devices. The compositions are effective visualization devices and reduce or even substantially eliminate the risk of ocular side effects when introduced into the eye relative to a visualization device composition including a pharmacologically effective amount of a therapeutic component, such as Kenalog® 40.

As noted above, the present compositions include a contrast component. Such contrast component is present in the compositions in an amount effective, when the composition is placed in a vitreous of an eye, (1) to be opaque, for example, to light, such as, and without limitation, visible light, or (2) to be visually distinct, for example, to the vitreous of the eye, such as in light, for example, and without limitation, visible light. The opaque/visually distinct character of the contrast component in the vitreous is important in allowing visualization, for example, and without limitation, direct visualization, of the vitreous of the eye.

In one embodiment, the contrast component, which is substantially pharmacologically inert or inactive in the eye in which the composition is placed, has a different refractive index (index of refraction) than both the carrier component and the vitreous into which the composition is placed. Without wishing to limit the invention to any particular theory of operation, it is believed that such different refractive indexes facilitate or even cause the contrast component to be opaque/visually distinct in the vitreous of the eye in which the composition is placed. The refractive indexes of the contrast component and the carrier component advantageously differ by at least about 0.05, for example, differ by at least about 0.15.

Any suitable contrast component may be employed in the present invention. The contrast component preferably is substantially non-toxic to the eye in which the composition is placed, as well as being substantially pharmacologically inert or inactive. The contrast component may be inorganic or organic, biodegradable or non-biodegradable, and soluble or insoluble in the composition, for example, inside or outside of the eye.

In one useful embodiment, the contrast component comprises a plurality of particles. Although the size of the particles can vary over a relatively wide range, such particles should be of a size to provide the desired opacity/visual distinctiveness, without injuring the tissues of the eye in which the composition is placed. Also, the size of the particles should be such as to cause no substantial or even significant interference with administering the composition to the eye.

The contrast component may advantageously be present as a plurality of particles having a maximum transverse dimension, e.g., diameter, of less than about 50 microns. Particles having a maximum transverse dimension in a range of about 1 micron or less or about 3 microns to about 10 microns or about 20 microns are very useful.

Among useful inorganic contrast components, for example, present as a plurality of particles, include, without limitation, talc, titanium oxide, zinc oxide and the like and mixtures thereof.

In a useful embodiment, the contrast component may be organic. For example, and without limitation, organic contrast components include solid lipid nanoparticles (SLNs), oils, such as in oil-in-water emulsions and the like, as well as other organic-containing materials which are compatible with or in the eye and are biodegradable and/or autoclavable and/or have sufficiently strong light scattering properties.

Among the oils that may be included in oil-in-water emulsions in accordance with the present invention include, without limitation, pharmaceutical grade oils, for example, without limitation, mineral oils, vegetable oils and other oils, such as soybean oil, safflower oil, caster oil, olive oil and the like and mixtures thereof. The oil or oils in the oil-in-water emulsions may be considered to be the contrast component in such compositions, while the water may be considered to be at least part of the carrier component.

The oil or oils are present in the oil-in-water emulsions in accordance with the present invention in an amount effective to provide sufficient opacity/visual distinctiveness to be effective as a visualization device for the vitreous or to aid in visualizing the vitreous. The amount of oil used may depend, and therefore vary based on, the specific oil employed, the other components of the composition, the particular application in which the composition is to be used and the like factors. The oil is often present in the emulsion in an amount of less than about 50% (w/v) of the emulsion. In one embodiment, the oil is present in an amount in a range of about 1% (w/v) or less or about 5% (w/v) to about 10% (w/v) or about 20% (w/v) or about 30% (w/v) or more.

Such oil-in-water emulsions may include one or more additional components. For example, emulsification components may often be employed in an amount effective to facilitate the formation and/or the maintenance of the oil-in-water emulsion. Such emulsification components may be nonionic or ionic, for example, anionic, in character. Examples of useful emulsification components include, without limitation, egg yolk phospholipids, polyoxyethylene-polyoxypropylene copolymers, for example block copolymers, such as those sold under the trademark Pluronic® and Tetronic®, fatty acid ethoxylates, for example, polyoxyethylene sorbitan fatty acid ester ethoxylates, such as polysorbate 20-80 and the like, polyoxyethylene stearates, medium chain triglycerides, crosslinked polyacrylic acids, such as those sold under the trademark Pemulen®, and the like and mixtures thereof. Such emulsification components advantageously are compatible or "friendly" with the eye and are biodegradable. The amount of emulsification component used may depend, and therefore vary based on, the specific emulsion desired, the other components of the composition, the particular application in which the composition is to be used and the like function. The emulsification components may be present in the oil-in-water emulsions in amounts ranging from about 0.1% (w/v) or less or about 0.2% (w/v) to about 2% (w/v) or about 5% (w/v) or more.

The oil-in-water emulsions of the present invention may include tonicity agents, for example and without limitation, nonionic tonicity agents, such as glycerol, mannitol and the like and mixtures thereof, buffer components, pH adjustor components, and the like and mixtures thereof.

In one embodiment, the contrast component comprises a polymeric material, for example, a polymeric material which comprises a plurality of particles. The polymeric material may be selected from homopolymers, copolymers, that is polymers which include units from 2, 3, 4, or more monomers, and the like and mixtures thereof. Examples of biodegradable polymeric materials which may be employed, in the present contrast components includes, without limitation, those selected from polylactides, polglycolides, poly(lactide-co-glycolides), polyanhydrides, poly(caprolactone), polycarbonates, polyarylates, polydioxanones, polyhydroxyalkanoates, polyphosphazenes, chitosan and the like and mixtures thereof.

Examples of non-biodegradable polymeric materials useful in the present contrast components include, without limitation, polyolefins, such as polyethylene, polypropylene, polystyrene and the like, ethylene/vinyl acetate copolymers, silicone polymers, such as polysiloxanes, e.g., polymethylsiloxanes, and the like, poly(vinylchloride) and the like and mixtures thereof. In one embodiment, the contrast component advantageously has a limited solubility in water, for example at 25°C. For example, the contrast component may have a solubility in water at 25°C of about 10 mg/ml or less or about 5 mg/ml or less, or about 1 mg/ml or less. Of course, the contrast component, as well as the present compositions should be ophthalmically acceptable, that is should have substantially no significant or undue detrimental effect on the eye structures or tissues.

The contrast component is present in the present compositions in an amount effective to provide sufficient opacity/visual distinctiveness when the composition is placed in the eye to facilitate and/or provide visualization of the vitreous body. The particular amount of contrast component to be used depends on the particular contrast component used, the carrier component used, the degree of opacity/visual distinctiveness desired in the vitreous of the eye, and the specific application in which the composition is to be used, as well as other factors. In one embodiment, the contrast component is present in an amount of at least about 0.000001% (w/v) or about 0.1% (w/v) or about 0.5% (w/v) of the composition. Advantageously the contrast component is present in an amount in a range of about 0.000005% (w/v) or about 0.1% (w/v) or about 0.5% (w/v) or about 1% (w/v) to about 5% (w/v) or about 10% (w/v) or about 15% (w/v) or more, for example in a range of about 0.5% (w/v) or about 1% (w/v) to about 10% (w/v), of the composition.

In one embodiment, the contrast component and the carrier component together form a composition in the form of an opaque solution or a colored solution. Thus, in certain embodiments, the contrast component is soluble in the carrier component of the present compositions. In these embodiments, the refractive index of the contrast component in such solutions may be different from the refractive index of the carrier component in such solutions and/or the refractive index of the opaque or colored solution may be different from the refractive index of the vitreous of the eye in which the solution is to be placed. Such opaque or colored solutions are useful as visualization devices in accordance with the present invention. One advantage of employing solutions as visualization devices in accordance with the present invention is that such compositions can be made free of resuspension components or emulsification components. Examples of such soluble contrast components for use with aqueous carrier components in the present compositions include, without limitation, sodium fluorescein, fluorescein isothiocyanate (FITC)-dextran, Food, Drug and Cosmetic (FD&C) dyes, such as Yellow 5, Red 3, Blue 1, Red 40, Orange B, Citrus Red 2, Yellow 6, Blue 2 and Green 3 dyes, and the like and mixtures thereof. The soluble contrast components may be present in widely varying amounts depending, for example and without limitation, on the specific contrast component employed. For example, in one embodiment, the soluble contrast component may be present in an amount of about 0.000001% (w/v) or less or about 0.000005% (w/v) to about 0.2% (w/v) or about 0.5% (w/v) or about 1.0% (w/v) or more of the composition.

The carrier component of the present composition may be selected from any suitable material effective to act as a carrier for the contrast component, for example, in the vitreous of an eye in which the composition is placed. Preferably, the carrier component is liquid and is ophthalmically acceptable in an eye in which the composition is placed.

In one useful embodiment, the carrier component is aqueous or aqueous-based, for example, and without limitation, an aqueous or aqueous-based liquid. The carrier component advantageously comprises a major amount, that is at least about 50% (w/v), of water.

The carrier components, for example, the aqueous carrier component, is advantageously ophthalmically acceptable and may include one or more conventional excipients useful in ophthalmic compositions.

The present compositions preferably include a major amount of liquid water. The present compositions may be, and are preferably, sterile, for example, prior to being used in the eye.

The present compositions preferably include at least one buffer component in an amount effective to control the pH of the composition and/or at least one tonicity component in an amount effective to control the tonicity or osmolality of the compositions. More preferably, the present compositions include both a buffer component and a tonicity component.

The buffer component and tonicity component may be chosen from those which are conventional and well known in the ophthalmic art.

Examples of such buffer components include, without limitation, acetate buffers, citrate buffers, phosphate buffers, borate buffers and the like and mixtures thereof. Useful tonicity components include, without limitation, salts, particularly sodium chloride, potassium chloride, and the like, other suitable ophthalmically acceptably tonicity components such as glycerol, mannitol and the like, and mixtures thereof.

The amount of buffer component employed preferably is sufficient to maintain the pH of the composition in an ophthalmically acceptable range, for example, in a range of about 6 to about 8, or about 7 to about 7.5. The amount of tonicity component employed preferably is sufficient to provide an ophthalmically acceptable osmolality to the present compositions. Such osmolality may be in a range of about 200 to about 400, or about 250 to about 350, mOsmol/kg. Advantageously, the present compositions are substantially isotonic.

In addition, the present composition may include an effective amount of resuspension component effective to facilitate the suspension or resuspension of the contrast component, such as the particles of contrast component in the present compositions. In some embodiments of the present compositions effective amounts of resuspension components are employed, for example, to provide an added degree of insurance that the contrast component particles remain in suspension, as desired and/or can be relatively easily resuspended in the present compositions.

Any suitable resuspension component may be employed in accordance with the present invention. Examples of such resuspension components include, without limitation, surfactants such as polyoxyethylene-polyoxypropylene copolymers, for example, block copolymers, such as those sold under the trademark Pluronic® and Tetronic®; fatty acid ethoxylates, for example, polyoxyethylene sorbitan fatty acid ester ethoxylates such as polysorbate 20-80 and the like; tyloxapol; sarcosinates; polyethoxylated castor oils, cellulosic derivatives having surfactant propertied, such as low molecular weight alkali metal, e.g., sodium, carboxymethyl cellulose; vitamin-based surfactants including, without limitation. Vitamin E tocopheryl polyethylene glycol succinates, such as Vitamin E tocopherol polyethylene glycol 1000 succinate (Vitamin E TPGS); other surfactants and the like and mixtures thereof.

The presently useful resuspension components are present, if at all, in the compositions in accordance with the present invention in an amount effective to facilitate suspending and/or resuspending the particles in the present compositions, for example, during manufacture of the compositions or thereafter. The specific amount of resuspension component employed may vary over a wide range depending, for example, on the specific resuspension component being employed, the specific composition in which the resuspension component is being employed and the like factors. Suitable concentrations of the resuspension component, if any, in the present compositions are often in a range of about 0.01% to about 5%, for example, about 0.02% or about 0.05% to about 3% (w/v) of the composition.

The present compositions may include one or more other components in amounts effective to provide one or more useful properties and/or benefits to the present compositions. For example, although the present compositions may be substantially free of added preservative components, in other embodiments, the present compositions include effective amounts of preservative components, preferably such components which are more compatible with or more "friendly" to the tissue in the posterior segment, for example, and without limitation, the retina, of the eye into which the composition, is placed relative to benzyl alcohol, for example, at identical or equivalent antimicrobial concentrations. Examples of such preservative components include, without limitation, benzalkonium chloride, chlorhexidine, PHMB (polyhexamethylene biguanide), methyl and ethyl parabens, hexetidine, chlorite components, such as stabilized chlorine dioxide, metal chlorites and the like, other ophthalmically acceptable preservatives and the like and mixtures thereof.

The concentration of the preservative component, if any, in the present compositions is a concentration effective to preserve the composition, and is often in a range of about 0.00001% to about 0.05% or about 0.1% (w/v) of the composition.

The viscosity of the present compositions may vary over a relatively wide range provided that such viscosity does not substantially or significantly interfere with the usefulness of the composition as a visualization device as described herein. In particular, the viscosity of the present compositions advantageously is sufficiently low to allow the compositions to readily diffuse to a desired extent into the vitreous body after being placed in the vitreous of an eye. For example, when it is desired to be able to visualize substantially the entire vitreous body, the present compositions are preferably such that the compositions effectively, e.g., for visualization purposes, disperse throughout the entire vitreous body in a time of about 5 minutes or less or about 2 minutes or less or about 1 minute or less, for example, about 10 to about 40 seconds, after the composition is placed in the vitreous. The viscosity of the present compositions when placed in the vitreous of an eye preferably is less than the viscosity of the vitreous material in which it is placed. Relatively low viscosities are useful in facilitating administering, for example, injecting, the compositions into the vitreous and/or facilitating the compositions defusing in the vitreous body to provide better or more complete visualization of the vitreous or vitreous body. In one embodiment, the present compositions have viscosities of about 50 cps or less, or about 30 cps or less or about 20 cps or less, for example, at 25°C. Viscosities in a range of about 1 cps or less to about 3 cps or about 5 cps or about 10 cps at 25°C are very useful.

The present compositions can be prepared using suitable blending/processing techniques, for example one or more conventional blending techniques. The preparation processing should be chosen to provide the present compositions in forms which are useful for placement or injection into the vitreous bodies of eyes of humans or animals. In one useful embodiment, a concentrated contrast component dispersion is made by combining the contrast component with water, and the excipients to be included in the final composition. The ingredients are mixed to disperse the contrast component and then autoclaved. Alternately, the contrast component powder may be gamma-irradiated before addition to form a sterile carrier. Components, if any, which are sensitive, for example, subject to degradation or inactivation, to elevated temperature and/or gamma-radiation, may be purchased sterile or sterilized by other conventional processing to which such components are not sensitive, for example, by filtering a dilute solution of the component or components followed by lyophilization to yield a sterile powder.

The sterile powder or powders are combined with water to make an aqueous concentrate. Under aseptic conditions, the concentrated contrast component dispersion is mixed and added as a slurry to the aqueous concentrate. Water is added in a quantity sufficient (q.s.) to provide the desired composition and the composition is mixed until homogenous.

The compositions may be packaged in bulk form. However, the present compositions, sterile and without added preservative component, advantageously are packaged in a single or unit dose format, that is in a sealed package holding a quantity of the composition sufficient only for a single use. In one embodiment, such compositions are packaged in a sealed syringe in an amount sufficient only for a single use.

Methods of using the present composition are provided and are included within the scope of the present invention. In general, such methods comprise placing, for example, injecting or otherwise administering, an effective amount of a composition in accordance with the present invention in a vitreous of an eye of a human or animal. In the vitreous, the composition is effective as a visualization device for the vitreous body. With such visualization device in place, a surgeon can conduct a surgical operation on the eye, for example, to remove at least a portion of the vitreous from the eye, effectively and more safely since the extent of the vitreous body can be directly visualized by the surgeon, for example, under visible light.

The placing step advantageously comprises intravitreal injecting of the composition. A syringe apparatus including an appropriately sized needle, for example, a 27 gauge needle or a 30 gauge needle, can be effectively used to inject the composition into the vitreous of an eye of a human or animal.

The following non-limiting Examples illustrate certain aspects of the present invention.

### EXAMPLES 1 to 3

A series of three (3) suspensions are prepared as follows.

A dry polymer powder is micronized (to about 10-20 microns diameter) with an Aljet mill (Model 00 Jet-0-Mizer, Fluid Energy, Telfors, PA). The micronized polymer powder is combined with an isotonic pH-buffered aqueous solutions to form a smooth, milky white suspension which is Sterrile, or sterilized by autoclaving or gamma-radiation. Each of such suspensions is placed or packaged in a sealed glass vial.

The suspensions have the following compositions:

| **INGREDIENT** | **CONCENTRATION, % w/v** | | |
|---|---|---|---|
| | **Example 1** | **Example 2** | **Example 3** |
| Polylactide | 7.5 | - | - |
| Poly(lactide-coglycolide) | - | 7.5 | |
| Poly(caprolactone) | - | - | 7.5 |
| Sodium Carboxymethyl-Cellulose (low Viscosity) ⁽¹⁾ | 1.5 | 1.5 | 1.5 |
| Polysorbate 80 ⁽²⁾ | 0.5 | 0.5 | 0.5 |
| Boric Acid | 0. 6 | 0.6 | 0.6 |
| Sodium Borate | 0.05 | 0.05 | 0.05 |
| Sodium Chloride | 0.39 | 0.39 | 0.39 |
| Purified Water | QS | QS | QS |

| | | | |
|---|---|---|---|
| ⁽¹⁾ Weight Average Molecular Weight of about 90,000; manufactured by Aqualon, Inc. ⁽²⁾ A commercially available material including one or more fatty acid ethoxylates. Such material is an effective resuspension component in the suspensions. | | | |

Each of these suspensions exhibits a viscosity below about 5 cps at 25°C. Upon standing, the dispersed polymer particles form a loose cake that is easily resuspended with several gentle inversions of the glass vial in which the suspension is placed. Each of the polymers used in this series of compositions is biodegradable or bioerodible.

### EXAMPLES 4 TO 7

Using a procedure substantially similar to that set forth in Examples 1 to 3, an additional series of four (4) smooth, milky white suspensions are prepared. Each of the sterile suspensions is placed in a sealed glass vial.

The suspensions have the following compositions:

| **INGREDIENT** | **CONCENTRATION, % w/v** | | | |
|---|---|---|---|---|
| | **Example 4** | **Example 5** | **Example 6** | **Example 7** |
| Polystyrene | 7.5 | | | |
| Ethylene/vinyl acetate copolymer | | 7.5 | | |
| Poly(dimethyl siloxane) | | | 7.5 | |
| Poly(vinyl chloride) | | | | 7.5 |
| Sodium Carboxymethyl-cellulose (low Viscosity) ⁽¹⁾ | 0.5 | 0.5 | 0.5 | 0.5 |
| Polysorbare 80 ⁽²⁾ | 0.5 | 0.5 | 0.5 | 0.5 |
| Sodium phosphate monobasic | 0.16 | 0.16 | 0.16 | 0.16 |
| Sodium phosphate dibasic | 0.76 | 0.76 | 0.76 | 0.76 |
| Sodium Chloride | 0.39 | 0.39 | 0.39 | 0.39 |
| Purified Water | QS | QS | QS | QS |

Each of these suspensions exhibits a viscosity below about 5 cps at 25°C. Upon standing, the dispersed polymer particles form a loose cake that is easily resuspended with several gentle inversions of the glass vial in which the suspension is placed. Each of the polymers used in this series or compositions is non-biodegradable.

### EXAMPLES 8 to 10

Using a procedure substantially similar to that set forth in Examples 1 to 3, a further series of three (3) smooth, milky white suspensions are prepared. In these suspensions the dry polymer powder is replaced by powders of talc, titanium oxide and zinc oxide, respectively. Thus, the solid particles in each of the suspensions of Examples 8 to 10 are inorganic in contrast to the organic particles in the suspensions of Examples 1 to 7.

Each of the sterile suspensions of Examples 8 to 10 is placed in a sealed glass vial.

The suspensions have the following compositions:

| **INGREDIENT** | **CONCENTRATION, % w/v** | | |
|---|---|---|---|
| | **Example 8** | **Example 9** | **Example 10** |
| Talc | 5.0 | | |
| Titanium oxide | | 5.0 | |
| Zinc oxide | | | 5.0 |
| Sodium Carboxymethyl-Cellulose (low Viscosity) ⁽¹⁾ | 1.5 | 1.5 | 1.5 |
| Polysorbate 80 ⁽²⁾ | 0.5 | 0.5 | 0.5 |
| Boric Acid | 0.6 | 0.6 | 0. 6 |
| Sodium Borate | 0.05 | 0.05 | 0.05 |
| Sodium Chloride | 0.39 | 0.39 | 0.39 |
| Purified Water | QS | QS | QS |

Each of these suspensions exhibits a viscosity below about 5 cps at 25°C. Upon standing, the dispersed particles form a loose cake that is easily resuspended with several gentle inversions of the glass vial in which the suspension is placed.

### EXAMPLES 11 AND 12

A series of two (2) compositions are prepared as follows.

Sodium fluorescein and FD&C Red 3 dye are selected. Sodium fluorescein and FD&C Red 3 dye are each dissolved in an isotonic, pH buffered aqueous solution. The compositions formed from the combinations of sodium fluorescein and FD&C Red 3 dye and the aqueous solutions are colored solutions. Each solution is sterile or is sterilized by sterile filtration. Each such solution is placed or packaged in a sealed glass vial.

These colored solutions have the following compositions:

| **INGREDIENT** | **CONCENTRATION, % w/v** | |
|---|---|---|
| | **Example 11** | **Example 12** |
| Sodium Fluorescein | 0.00001 | |
| FD&C Red 3 dye | | 0.1 |
| Sodium Phosphate, Monobasic | 0.04 | 0.04 |
| Sodium Phosphate, Dibasic | 0.3 | 0.3 |
| Sodium Chloride | 0.63 | 0.63 |
| Water for Injection | QS | QS |

Each of these solutions exhibits a viscosity at about 1 cps at 25°C. Since these are solutions, no resuspension components are required.

### EXAMPLES 13 and 14

Two pharmaceutical grade vegetable oils are selected: soybean oil and safflower oil. Both oils are composed of long chain triglycerides and are suitable for injection into the vitreous of an eye.

A submicron oil-in-water emulsion is prepared from each of the oils as follows.

Pharmaceutical grade, fractionated egg yolk phospholipids are dispersed in hot (80°C) soybean oil or the safflower oil, as the case may be, using a water-jacketed stainless steel vessel and a high shear, rotorstator overhead mixer. After a uniform, slightly turbid dispersion is formed, hot (80°C) water is added slowly in a thin stream while continuing high shear mixing. Next, a non-ionic tonicity agent, glycerol or mannitol, as the case may be, is blended into the coarse emulsion, and sufficient sodium hydroxide added to raise the pH to 7.5-8.5.

The coarse emulsion is then homogenized at elevated temperature (50-60°C) in a Manton-Gaulin piston-valve type homogenizer operated at about 8,000-10,000 psig. After sufficient processing time, a submicron emulsion is produced which is filled into pharmaceutical glass bottles, stoppered and capped, and then sterilized in a steam autoclave. The emulsions appear opaque and milky white due to their strong light-scattering characteristics.

These biodegradable, oil-in-water emulsions have the following compositions:

| **INGREDIENT** | **CONCENTRATION, % w/v** | |
|---|---|---|
| | **Example 13** | **Example 14** |
| Soybean oil | 20.0 | - |
| Safflower oil | - | 20.0 |
| Egg yolk phospholipids, fractionated | 1.2 | 1.2 |
| Glycerol | 2.25 | - |
| Mannitol | - | 4.5 |
| Sodium hydroxide | pH adjust to 7.5-8.5 | pH adjust to 7.5-8.5 |
| Water | QS | QS |

Each of these emulsions exhibits a viscosity below about 10 cps at 25°C. Since these submicron dispersions are stable for at least two years at room temperature, no resuspension components are required.

### EXAMPLES 15-28

Each of the sterile compositions 1-14 is packaged in a sealed single use or single dose package, for example containing about 1.0 ml of the composition. The packaged compositions are now ready for use as visualization devices.

### EXAMPLE 29

The composition of Example 1 is used as a visualization device as follows.

A human patient is readied for vitrectomy surgery. In order to facilitate the surgeon's ability to visualize the vitreous or vitreous body during this surgery, the single dose package of the composition of Example 1, that is Composition 1, is employed. The package is inverted several times to provide that the suspension therein is substantially uniform. The suspension is then introduced into an injection device, for example, a syringe and an appropriately sized needle, for example, a 27 gauge needle or a 30 gauge needle. Using the loaded injection device, the suspension is placed in the vitreous of the patient's eye.

The suspension in the vitreous of the patient's eye allows the surgeon, through direct visualization under visible light, to clearly see or visualize the vitreous.

The surgeon than proceeds with and completes the surgery successfully. Since the suspension includes no pharmacologically active or effective component, the patient is advantageously subjected to no unwanted drug side effects and no risk of such drug side effects. In addition, since the suspension is sterile and includes no added preservative, the patient is protected against side effects or unwanted reactions from such added preservatives.

### EXAMPLES 30 TO 38

The composition of each of Examples 2 to 10 (Compositions 2 to 10) is used as a visualization device in a manner substantially similar to that as described in Example 29 with regard to Composition 1. Substantially similar results and advantages as set forth in Example 29 with regard to Composition 1 are obtained in each of Examples 30 to 38 with regard. to Compositions 2 to 10, respectively.

### EXAMPLES 39 AND 40

The composition of each of Examples 11 and 12 (Compositions 11 and 12) is used as a visualization device in a manner substantially similar to that as described in Example 29 with regard to Composition 1. Since Compositions 11 and 12 are colored solutions, there is no need to invert the package several times as in Example 29. Substantially similar results and advantages as set forth in Example 29 with regard to Composition 1 are obtained in each of Examples 39 and 40 with regard to Compositions 11 and 12.

### EXAMPLES 41 AND 42

The composition of each of Examples 13 and 14 (Compositions 13 and 14) is used as a visualization device in a manner substantially similar to that described in Example 29 with regard to Composition 1. Since Compositions 13 and 14 are stable oil-in-water emulsions, there is no need to invert the package several times as in Example 29. Substantially similar results and advantages as set forth in Example 29 with regard to Composition 1 are obtained in each of Examples 41 and 42 with regard to Compositions 13 and 14.

### EXAMPLES 43 AND 44

Two compositions were selected for testing. Composition 43 was substantially identical to Composition 1 except that Composition 43 included 2.5% w/v of poly(lactide-coglycolide)(PLGA) particles. Composition 44 was a sample of the commercially available product Kenelog® 40 and included an aqueous suspension of triamcinolone acetonide (TA) (4% (w/v)) and benzyl alcohol (1% w/v) as a preservative.

The specific purpose of this study was to compare Compositions 43 and 44 as visualization agents of the vitreous cortex during standard 25 gauge pars plana vitrectomy procedures and to assess residual pharmacological effects following the procedure in pigmented rabbits. After the standard procedure was performed on each of ten rabbits, five (5) rabbits were administered 0.1 ml of Composition 43 and five (5) rabbits were administered 0.1 ml of Composition 44 to visualize the vitreous cortex. The composition (Composition 43 or 44) was removed via vitrectomy and/or a soft tip needle.

During the vitrectomy procedure, Composition 43 (2.5% PLGA) gave approximately equivalent opacity as did Composition 44 (4% TA), and these suspensions worked equally well to visualize the vitreal cortex.

Forty-eight (48) hours post-surgery, VEGF was injected intravitreally to induce retinal vasculopathy and to aid in the observation of any pharmacological activities induced by residual levels of Compositions 43 and 44. Measurements were made at baseline (pre-VEGF) and at 48 hours post-VEGF administration using retina color fundus imaging, fluorescein angiography, and fluorophotometry to assess leakage of retinal vasculature.

Measurements by fluorescein angiography and fluorophotometry showed significantly less retinal vessel leakage in the rabbits treated with Composition 44 (4 % TA) compared to the rabbits treated with Composition 37 (2.5% PLGA).

This study demonstrated that Compositions 43 and 44 were equally effective as visualization aids during pars plana vitrectomy. However, the use of Composition 44 (4% TA) resulted in significant residual pharmacological activity in the posterior chamber for at least four days after the surgical procedure. In contrast, Composition 43 (2.5% PLGA), in accordance with the present invention showed substantially no residual pharmacological activity. Further, it should be noted, that since Composition 43 is preservative free, the use of this composition as a visualization aid avoids the risk of adverse reactions to the presence of preservatives, such as the benzyl alcohol in Composition 44.

The present compositions and methods advantageously provide visualization of the vitreous, for example, during surgery, such as vitrectomy surgery, without exposing the patient to unwanted side effects or the risk of unwanted side effects caused by the presence of pharmacologically active or effective agents. The contrast component in the present compositions may be organic or inorganic, biodegradable or non-biodegradable and soluble or insoluble in the composition. In one very useful embodiment, the compositions are substantially free of added preservatives. In short the present invention provides substantial benefits relative to prior art visualization devices.

Some preferred embodiments (EM) of the invention are listed under points 1 to 45 below.
1. An ophthalmic composition effective as a visualization device in a vitreous of an eye comprising:
   a contrast component in an amount effective when the composition is placed in a vitreous of an eye (1) to be opaque or (2) to be visually distinct; and
   a carrier component combined with the contrast component and being effective to act as a carrier for the contrast component, the composition being substantially free of any therapeutic component effective to have pharmacological activity in an eye in which the composition is placed.
2. The composition of EM 1 wherein the contrast component and the carrier component have different refractive indexes.
3. The composition of EM 1 wherein the contrast component comprises a plurality of particles.
4. The composition of EM 3 wherein the plurality of particles have maximum transverse dimensions of less than about 50 microns.
5. The composition of EM 1 which is in the form of an aqueous suspension.
6. The composition of EM 5 wherein the carrier component includes at least one resuspension component in an amount effective to provide enhanced suspension maintenance or to provide enhanced ability to the composition to reform as a suspension relative to an identical composition without the at least one suspension aid component.
7. The composition of EM 6 wherein the at least one resuspension component is selected from the group consisting of surfactant components, wettability components and mixtures thereof.
8. The composition of EM 1 wherein the contrast component is soluble in the composition at conditions outside an eye.
9. The composition of EM 1 wherein the contrast component is insoluble in the composition at conditions outside an eye.
10. The composition of EM 1 wherein the contrast component and the carrier component form a colored solution.
11. The composition of EM 1 comprising an oil-in-water emulsion.
12. The composition of EM 1 which is substantially non-toxic in an eye in which the composition is placed.
13. The composition of EM 1 wherein the contrast component is inorganic.
14. The composition of EM 1 wherein the contrast component is organic.
15. The composition of EM 1 wherein the contrast component is biodegradable.
16. The composition of EM 1 wherein the contrast component comprises a polymeric material.
17. The composition of EM 16 wherein the polymeric material comprises a plurality of particles.
18. The composition of EM 16 wherein the polymeric material comprises a polymer selected from the group consisting of polylactides, polyglycolides, poly(lactide-co-glycolides), polyanhydrides, anhydride/ ether copolymers poly(caprolactone), polycarbonates, polyarylates, polydioxanones, polyhydroxyalkanoates, polyphosphazenes, chitosan and mixtures thereof.
19. The composition of EM 16 wherein the polymeric material comprises a polymer selected from the group consisting of polyolefins, ethylene/vinyl acetate copolymers, silicone polymers, poly(vinyl chloride) and mixtures thereof.
20. The composition of EM 1 wherein the carrier component is aqueous.
21. The composition of EM 1 wherein the carrier component includes at least one additional component in an amount effective to enhance the ocular compatibility of the composition relative to an identical composition without the at least one additional component.
22. The composition of EM 27 wherein the carrier component is aqueous and the at least one additional component is selected from the group consisting of buffer components, tonicity components, resuspension components, emulsification components and mixtures thereof.
23. The composition of EM 1 which is sterile.
24. The composition of EM 1 which includes no preservative component.
25. The composition of EM 1 in a single use format.
26. A method of providing visualization of a vitreous body of an eye, the method comprising:
   placing an effective amount of a composition in a vitreous body of an eye, the composition comprising a contrast component in an amount effective when the composition is so placed (1) to be opaque or (2) to be visually distinct, and a carrier component combined with the contrast component and being effective to act as a carrier for the contrast component, the composition being free of any therapeutic component effective to have pharmacological activity in the eye when the composition is so placed.
27. The method of EM 26 wherein the contrast component comprises a plurality of particles.
28. The method of EM 35 wherein the contrast component is biodegradable.
29. The method of EM 35 wherein the contrast component comprises a polymeric material.
30. The method of EM 35 wherein the carrier component is aqueous.
31. The method of EM 26 wherein the composition is in the form of an aqueous suspension.
32. The method of EM 26 wherein the composition comprises an oil-in-water emulsion.
33. The method of EM 26 wherein the contrast component and carrier component together form a colored solution.
34. The method of EM 26 wherein the carrier component includes at least one additional component in an amount effective to enhance the ocular compatibility of the composition relative to an identical 1 composition without the at least one additional component.
35. The method of EM 26 wherein the composition includes no preservative component.
36. A method of removing at least a portion of a vitreous from an eye, the method comprising:
   placing an effective amount of a composition in a vitreous body of an eye, the composition comprising a contrast component in an amount effective when the composition is so placed (1) to be opaque or (2) to be visually distinct, and a carrier component combined with the contrast component and being effective to act as a carrier for the contrast component, the composition being free of any therapeutic component effective to have pharmacological activity in the eye when the composition is so placed; and, thereafter,
   removing at least a portion of the vitreous from the eye.
37. The method of EM 36 wherein the removing step is conducted using direct visualization of the vitreous body.
38. The method of EM 36 wherein the contrast component comprises a plurality of particles.
39. The method of EM 36 wherein the contrast component is biodegradable.
40. The method of EM 36 wherein the contrast component comprises a polymeric material.
41. The method of EM 36 wherein the carrier component is aqueous.
42. The method of EM 41 wherein the carrier component includes at least one additional component in an amount effective to enhance the ocular compatibility of the composition relative to an identical composition without the at least one additional component.
43. The method of EM 36 which is in the form of an aqueous suspension.
44. The method of EM 36 wherein the composition comprises an oil-in-water emulsion.
45. The method of EM 36 wherein the contrast component and carrier component together form a colored solution.

While this invention has been described with respect to various specific examples and embodiments, it is to be understood that the invention is not limited thereto and that it can be variously practiced within the scope of the following claims.

## Claims

1. An ophthalmic composition comprising:
an organic contrast component which when placed in a vitreous of an eye (1) is opaque or (2) is visually distinct; and
a carrier component combined with the contrast component, the composition being substantially free of any therapeutic component having pharmacological activity in an eye in which the composition is placed.

2. The composition of claim 1 wherein the contrast component comprises a polymeric material.

3. The composition of claim 2 wherein the polymeric material comprises a plurality of particles.

4. The composition of claim. 3 wherein the plurality of particles have maximum transverse dimensions of less than about 50 microns.

5. The composition of claim 2 wherein the polymeric material comprises a polymer selected from the group consisting of polylactides, polyglycolides, poly(lactide-co-glycolides), polyanhydrides, anhydride/ ether copolymers poly(Caprolactone), polycarbonates, polyarylates, polydioxanones, polyhydroxyalkanoates, polyphosphazenes, chitosan and mixtures thereof.

6. The composition of claim 2 wherein the polymeric material comprises a polymer selected from the group consisting of polyolefins, ethylene/vinyl acetate copolymers, silicone polymers, poly(vinyl chloride) and mixtures thereof.

7. The composition of claim 1 wherein the contrast component and the carrier component have different refractive indexes.

8. The composition of claim 1 which is in the form of an aqueous suspension.

9. The composition of claim 8 wherein the carrier component includes at least one resuspension component selected from the group consisting of surfactant components, wettability components and mixtures thereof.

10. The composition of claim 1 wherein the contrast component is soluble in the composition at conditions outside an eye.

11. The composition of claim 1 wherein the contract component is insoluble in the composition at conditions outside an eye.

12. The composition of claim 1 wherein the contrast component and the carrier component form a colored solution.

13. The composition of claim 1 comprising an oil-in-water emulsion.

14. Use of the ophthalmic composition according to any of claims 1-13 for providing visualization of a vitreous body of an eye.

15. The ophthalmic composition according to any of claims 1-13 for use in a method of removing at least a portion of a vitreous from an eye.
